# EUROPEAN PATENT SPECIFICATION

(11) **EP 4 074 307 B1**
(45) Date of publication and mention of the grant of the patent: **12.11.2025**
(21) Application number: 21168880.9
(22) Date of filing: 16.04.2021
(51) Int. Cl.: A61K 9/107, A61K 9/14, A61K 31/352, A61K 47/10, A61K 47/32, A61K 47/38, A61K 47/22, A61K 47/02

(54) **FORMULATIONS OF CANNABINOIDS**
FORMULIERUNGEN VON CANNABINOIDEN
FORMULATIONS DE CANNABINOÏDES

(43) Date of publication of application: 19.10.2022
(73) Proprietor: ADD Advanced Drug Delivery Technologies, Ltd., 4133 Pratteln (CH)
(72) Inventor: Nowak, Reinhard, 79589 Binzen (DE); Nowak, Mirko, 79540 Lörrach (DE); Nowak, Jesko Jay, 79540 Lörrach (DE); Dr. Pöllinger, Norbert, 79379 Müllheim (DE)
(74) Representative: Breuer, Markus

(56) References cited:
- EP-A1- 2 609 912
- WO-A1-2019/159174
- CN-A- 111 991 355
- GB-A- 2 584 341

## Description

### Field of the Invention

The present invention relates to formulations of cannabinoids, in particular of cannabidiol, as well as methods for preparing such formulations. The invention in particular provides solid formulations for oral administration of cannabinoids, in particular of cannabidiol.

### Background of the Invention

Cannabinoids and in particular cannabidiol (CBD) have been considered as drugs. There is evidence that cannabinoids can be beneficial for treating a number of clinical conditions, including pain, inflammation, epilepsy, sleep disorders, indication of multiple sclerosis, anorexia, and schizophrenia (N. Bruni et al., Cannabinoid Delivery Systems for Pain and Inflammation Treatment. Molecules 2018, 23, 2478).

While the use of cannabinoids in various indications has been suggested, but so far only limited applications received market authorisation.

Cannabinoids, in particular cannabidiol, are difficult to formulate due to their highly lipophilic nature. Low and variable bioavailability of cannabinoids, in particular upon oral administration, hampers effective clinical use of these compounds.

In fact, cannabinoids are highly lipophilic molecules (log P 6-7) with very low water solubility (2-10 µg / ml). The log P is the decimal logarithm of the n-octanol/water partition coefficient. The partition coefficient can be determined experimentally. Values typically refer to room temperature (25°C). The partition coefficient can also be roughly calculated from the molecular structure.

In addition to poor solubility, cannabinoids, in particular CBD, are subject to high first-pass metabolism, which further contributes to poor systemic availability after oral administration.

Various formulations of cannabinoids have been suggested.

Due to the high lipophilicity of cannabinoids, salt formation (*i.e*. pH adjustment), cosolvency (*e.g.* ethanol, propylene glycol, PEG400), micellization (*e.g.* Polysorbate 80, Cremophor-ELP), emulsification including micro and nano emulsification, complexation (*e.g.* cyclodextrins) and encapsulation in lipid-based formulations (*e.g*. liposomes) are among the formulation strategies considered in the prior art. Nanoparticle systems have also been proposed (N. Bruni *et al., loc. cit.*)*.*

Various solid oral dosage forms have been proposed in the patent literature, for example in WO 2008/024490 A2 and in WO 2018/035030 A1. These documents do not contain data on release behaviour, so the practical suitability of the proposed forms for the administration of cannabinoids remains unclear.

WO 2015/065179 A1 describes compressed tablets which, in addition to cannabidiol, contain lactose and sucrose fatty acid monoesters. Further solid cannabinoid formulations are disclosed in WO 2019/159174 A1, GB 2 584 341 A, EP 2 609 912 A1 and CN 111 991 355 A.

Dronabinol (Δ9-THC) is marketed in the form of capsules (Marinol^{®}) and as an oral solution (Syndros^{®}). The Marinol^{®} capsules are soft gelatine capsules containing the active ingredient in sesame oil.

The drug product Sativex^{®} containing nabiximols is a mouth spray that is sprayed onto the inside of the cheek.

Self-emulsifying drug delivery systems (SEDDS) which are mixtures of oils, surfactants and optionally contain hydrophilic solvents have also gained interest in an approach to improve the oral bioavailability of certain cannabinoids (K. Knaub *et al.* (2019). A Novel Self-Emulsifying Drug Delivery System (SEDDS) Based on VESIsorb^{®} Formulation Technology Improving the Oral Bioavailability of Cannabidiol in Healthy Subjects. Molecules, 24(16), 2967). Upon contact with an aqueous phase, such as gastric or intestinal fluids, SEDDS spontaneously emulsify under conditions of gentle agitation.

VESIsorb^{®}, a self-emulsifying drug delivery formulation technology developed by Vesifact AG (Baar, Switzerland) has shown increased oral bioavailability of certain lipophilic molecules.

The preparation Epidiolex^{®} recently approved by the US-FDA as an orphan drug for the treatment of certain forms of epilepsy is provided in the form of an oral solution that in addition to the active ingredient cannabidiol contains the excipients absolute ethanol, sesame oil, strawberry aroma and sucralose.

Notwithstanding all these proposals, however, there is still a need for improved dosage forms for cannabinoids, such as cannabidiol, in particular for solid oral dosage forms.

### Summary of the Invention

The present invention provides a pharmaceutical formulation in the form of a solid dispersion, wherein the solid dispersion comprises in admixture a cannabinoid, an amphiphilic block copolymer as a solubilizer and a water-soluble film former, according to claim 1.

The amphiphilic block copolymer is preferably a block copolymer containing at least one polyoxyethylene block and at least one polyoxypropylene block, such as a poloxamer. The water-soluble film former is preferably polyvinylpyrrolidone or hydroxypropylmethyl cellulose.

The above components are present in a weight ratio cannabinoid: amphiphilic block copolymer : water soluble film former of 1 : 0.16 - 0.36 : 0.08 - 0.28, more preferably 1 : 0.21 - 0.31 : 0.13 - 0.23.

The formulation preferably contains an antioxidant, in particular ascorbyl palmitate.

The cannabinoid is in particular cannabidiol (2-[(1R,6R)-3-methyl-6-(1-methylethenyl)-2-cyclohexen-1-yl]-5-pentyl-1,3-benzenediol).

A method for preparing a cannabinoid containing formulation according to the invention comprises the steps of (i) preparing a liquid composition comprising the cannabinoid, the amphiphilic block copolymer and a solvent capable of at least partially dissolving the cannabinoid and the amphiphilic block copolymer; (ii) introducing the liquid composition into a fluid bed granulator; (iii) removing solvent to obtain a solid dispersion in particulate form; and (iv) recovering the solid dispersion in particulate form from the fluid bed granulator.

Further objectives and their solution can be concluded from the detailed description of the invention below.

### Detailed Description of the Invention

### Active Ingredients

Cannabinoids are a heterogeneous group of pharmacologically active substances that have an affinity for the so-called cannabinoid receptors. The cannabinoids include, for example, tetrahydrocannabinol (THC) and the non-psychoactive cannabidiol (CBD). Cannabinoids can be both phytocannabinoids and synthetic cannabinoids.

Phytocannabinoids are a group of about 70 terpenophenolic compounds (V.R. Preedy (ed.), Handbook of Cannabis and Related Pathologies (1997)). These compounds typically contain a monoterpene residue that is attached to a phenolic ring and has a C₃-C₅ alkyl chain that is in the meta position to the phenolic hydroxyl group.

A preferred group of cannabinoids are tetrahydrocannabinols with the following general formula (1): 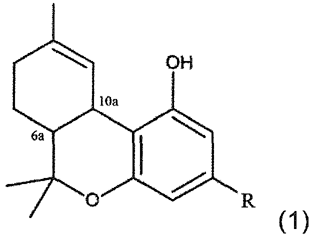 wherein R is selected from among C₁-C₂₀-alkyl, C₂-C₂₀-alkenyl or C₂-C₂₀-alkynyl, and optionally has one or more substituents.

In a further preferred group of compounds of the above general formula (1), R is selected from among C₁-C₁₀-alkyl or C₂-C₁₀-alkenyl, and optionally has one or more substituents.

In particular, in formula (1) R is an alkyl radical with the formula C₅H₁₁.

Compounds of general formula (1) can be present in the form of stereoisomers. The centres 6a and 10a preferably each have the R configuration.

The tetrahydrocannabinol is in particular Δ9-THC with the chemical name (6aR,10aR)-6,6,9-trimethyl-3-pentyl-6a, 7,8,10a-tetrahydro-6H-benzo[c]chromene-1-ol. The structure is reflected by the following formula (2):

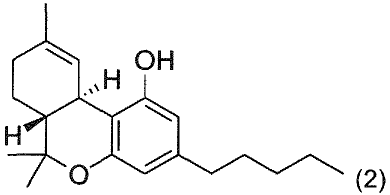

Another preferred group of cannabinoids are cannabidiols with the following general formula (3): 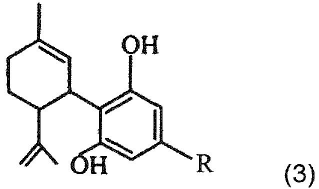 wherein R is selected from among C₁-C₂₀-alkyl, C₂-C₂₀-alkenyl or C₂-C₂₀-alkynyl, and optionally has one or more substituents.

In a further preferred group of compounds having the general formula (3) above, R is selected from among C₁-C₁₀-alkyl or C₂-C₁₀-alkenyl, and optionally has one or more substituents.

In particular, R in formula (3) is an alkyl radical with the formula C₅H₁₁.

The cannabidiol is in particular 2-[(1R,6R)-3-methyl-6-(1-methylethenyl)-2-cyclohexen-1-yl]-5-pentyl-1,3-benzenediol. In the present specification, if the term cannabidiol or its abbreviation CBD is used, this particular compound is meant, unless stated otherwise.

CBD is a major constituent of *Cannabis sp.* - besides the psychotropic Δ9-THC. The psychotropic effect of THC is mediated by the cannabinoid receptor CB1 that is mainly expressed on neurons. In contrast to THC, CBD is a peripherally and centrally acting compound without psychotropic activity.

According to the invention, a combination of Δ9-THC ((6aR, 10aR)-6,6,9-trimethyl-3-pentyl-6a,7,8,10a-tetrahydro-6H-benzo[c]chromen-1-ol) and CBD (2-[(1R,6R)-3-methyl-6-(1-methylethenyl)-2-cyclohexen-1-yl]-5-pentyl-1,3-benzenediol) can be used.

Another preferred group of cannabinoids are cannabinols with the following general formula (4): 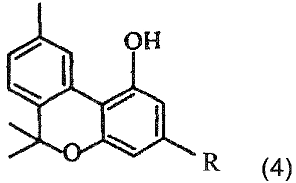 wherein R is selected from among C₁-C₂₀-alkyl, C₂-C₂₀-alkenyl or C₂-C₂₀-alkynyl, and optionally has one or more substituents.

In a further preferred group of compounds having the general formula (4) above, R is selected from among C₁-C₁₀-alkyl or C₂-C₁₀-alkenyl, and optionally has one or more substituents.

In particular, in formula (4) R is an alkyl radical having the formula C₅H₁₁.

The cannabinol is especially 6,6,9-trimethyl-3-pentyl-6*H*-dibenzo[*b,d*]pyran-1-ol.

According to the invention, cannabinoids or cannabinoid mixtures of hemp extracts can also be used.

For example, Nabiximols is a plant extract mixture used as a drug of the leaves and flowers of the hemp plant (Cannabis sativa L.) with standardized contents of tetrahydrocannabinol (THC) and cannabidiol (CBD).

Synthetic cannabinoids can also be used.

These include 3-(1,1-dimethylheptyl)-6,6a,7,8,10,10a-hexahydro-1-hydroxy-6,6-dimethyl-9*H*-dibenzo[*b,d*]pyran-9-one. This compound contains two stereogenic centres. The drug nabilone is a 1:1 mixture (racemate) of the (6a*R*,10a*R*) form and the (6a*S*,10a*S*) form. Nabilone is a preferred cannabinoid according to the invention.

Another example of a synthetic cannabinoid is JWH-018 (1-naphthyl-(1-pentylindol-3-yl)methanone).

The use of cannabinoids, in particular of cannabidiol, is based on their pharmacodynamic properties. Cannabinoid receptors include CB1, which is predominantly expressed in the brain, and CB2, which is primarily found on the cells of the immune system. The fact that both CB1 and CB2 receptors have been found on immune cells suggests that cannabinoids play an important role in the regulation of the immune system. Independent of this finding, several studies show that cannabinoids downregulate cytokine and chemokine production and, in some models, upregulate T-regulatory cells (Tregs) as a mechanism to suppress inflammatory responses. The endocannabinoid system is also involved in immunoregulation.

### Galenics

The present invention provides a pharmaceutical formulation which is a solid dispersion comprising a cannabinoid, in particular cannabidiol. As further detailed below, solid dosage forms for oral administration showing satisfactory bioavailability can be obtained in this way. Dosage forms according to the present invention also show a reduced food effect.

A highly lipophilic cannabinoid, like the almost water insoluble CBD, is combined with an amphiphilic block copolymer and with a water-soluble film former to form a solid dispersion. Incorporating a combination of a solubilizer and a water-soluble film former into the formulation allows adjusting the release rate of the cannabinoid.

The solid dispersion comprising a cannabinoid, in particular cannabidiol, an amphiphilic block copolymer and a water-soluble film former leads to the formation of micelles upon contact with water or other aqueous media, such as gastrointestinal fluids. The micelles are essentially formed from the drug substance, surrounded by the solubilizing excipients (see Fig. 1).

One aspect of the invention is accordingly a micellar composition comprising an aqueous phase in which micelles are dispersed, which micelles comprise a cannabinoid, in particular cannabidiol, and solubilizing excipients, in particular the amphiphilic block copolymer and the water-soluble film former.

The amphiphilic block copolymer present in the formulations of the present invention acts as a solubilizer. The reference to an amphiphilic block copolymer includes the possibility that more than one such copolymer is present.

The cannabinoid and the amphiphilic block copolymer are present in a weight ratio cannabinoid: amphiphilic block copolymer of 1 : 0.16 - 0.36, more preferably 1 : 0.21 - 0.31.

The amphiphilic block copolymers are solid at ambient temperature.

They have surfactant properties and, if used in appropriate concentration ranges in aqueous media, in particular water, can form micellar solutions.

In particular block copolymers containing at least one polyoxyethylene block and at least one polyoxypropylene block can be used.

Preferred block copolymers are poloxamers. Poloxamers are block copolymers whose molecular weights range from 1,100 to over 14,000. Different poloxamers differ only in the relative amounts of propylene and ethylene oxides added during manufacture. Poloxamers have the following general formula:

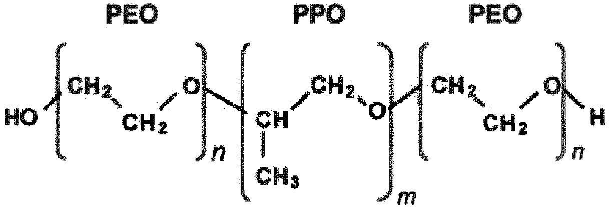

In this general formula, n designates the number of polyoxyethylene units, m designates the number of polyoxypropylene units.

In one embodiment, the solubilizer is Poloxamer 188 (Kolliphor P188; former brand name Lutrol F 68) / BASF; CAS No.: 9003-11-6).

Kolliphor P188 is a polyoxyethylene-polyoxypropylene block copolymer of the above general formula wherein n is approximately 79 and m is approximately 28.

Kolliphor P188 is available as a white to slightly yellowish waxy substance in the form of micropearls having a melting point of 52 - 57°C. It meets the requirements of Ph.Eur., USP / NF for Poloxamer 188.

As a further excipient, the formulations of the present invention contain a water-soluble film former. The reference to a water-soluble film former again includes the possibility that a combination of two or more such film formers is used.

The cannabinoid and the water soluble film former are present in a weight ratio cannabinoid : water soluble film former of 1 : 0.08 - 0.28, more preferably 1 : 0.13 - 0.23.

The water-soluble film former acts as a polymeric binder and additional solubilizer in the present formulation.

Examples of suitable water-soluble film formers are methyl cellulose (MC), hydroxypropyl methyl cellulose (HPMC), hydroxypropyl cellulose (HPC), hydroxyethyl cellulose (HEC), sodium carboxymethyl cellulose (Na-CMC) and polyvinyl pyrrolidone (PVP).

A preferred film former is PVP, in particular PVP K30 (such as Kollidon^{®} 30).

Another preferred film former is hydroxypropylmethyl cellulose (HPMC), in particular low-viscosity HPMC, such as HPMC with a viscosity of a 2% (w/w) aqueous solution at 20°C of 6 mPa·s or less.

The above discussed components are present in a weight ratio cannabinoid (in particular cannabidiol) : amphiphilic block copolymer: water soluble film former (polyvinylpyrrolidone) of 1 : 0.16 - 0.36 : 0.08 - 0.28, more preferably 1 : 0.21 - 0.31 : 0.13 - 0.23.

It is in particular considered to further include an antioxidant or a combination of antioxidants to protect the cannabinoid, in particular cannabidiol, from oxidation. Cannabinoids, in particular cannabidiol, are susceptible to oxidation. For instance, cannabidiol can be oxidized to monomeric and dimeric hydroxyquinones. The oxidation can lead to discoloration.

The oxidation can not only occur by molecular oxygen, but also by peroxides which may be introduced into the formulation by one or more of the excipients used.

Useful antioxidants which may be included into the formulation encompasses ascorbyl palmitate, alpha-tocopherol, butylhydroxytoluol (BHT, E321), butylhydroxyanisol (BHA, E320), ascorbic acid, and ethylenediaminetetraacetic acid (EDTA) sodium.

Ascorbyl palmitate is a preferred antioxidant. It can effectively suppress discoloration by oxidation.

The antioxidant is typically used in an amount of 0.5 to 2.5 wt%, preferably of 0.8 to 2 wt%, in particular 1.0 to 1.8 wt%, relative to the amount of the cannabinoid (in particular cannabidiol).

Other excipients may be present in addition.

In a preferred embodiment, the formulation contains in addition a diluent. Diluents (or fillers) as typically used in solid oral dosage forms can be employed. A preferred diluent is microcrystalline cellulose (such as Avicel^{®} PH 101).

Active ingredient and diluent are typically present in a weight ratio cannabinoid (in particular cannabidiol) : diluent (in particular microcrystalline cellulose) of 1:0.5 - 2.7, preferably 1:0.9 - 2.3, in particular 1:1.3 - 1.9.

In a still further embodiments, silicon dioxide (such as Syloid^{®} 244 FP Silica) and/or colloidal silicon dioxide (such as Aerosil^{®} 200) are included in the formulation, in particular to serve as moisture adsorbents.

Active ingredient and total silicon dioxide components are typically present in a weight ratio cannabinoid (in particular cannabidiol) : total amount of all silicon dioxide components of 0.14 - 0.44, preferably 0.19 - 0.39, in particular 0.24 - 0.34.

While formulations according to the present invention are not limited to those containing the above discussed excipients, the formulations are preferably free or essentially free of triglycerides. Essentially free means that the formulation contains less than 5 % by weight, relative to all components, of triglycerides.

The solid dispersion is preferably free or essentially free of triglycerides. Essentially free means that the formulation contains less than 5 % by weight, relative to all components, of triglycerides.

Further, the solid dispersion is preferably free or essentially free of mono- and diglycerides. Essentially free means that the formulation contains less than 5 % by weight, relative to all components, of mono- and diglycerides.

Still further, the solid dispersion is preferably free or essentially free of fatty acids. Essentially free means that the formulation contains less than 5 % by weight, relative to all components, of fatty acids.

Preferably, the total amount of mono-, di- and triglycerides and fatty acids is less than 5 % by weight, relative to all components.

The present pharmaceutical formulations in the form of solid dispersions can be obtained by wet granulation techniques. The granulation can be carried out in a blender. Preferably, fluid bed granulation technology can be used.

According to the present invention, a method for preparing a cannabinoid containing formulation comprises the steps of (i) preparing a liquid composition comprising the cannabinoid, the amphiphilic block copolymer and a solvent capable of at least partially dissolving the cannabinoid and the amphiphilic block copolymer; (ii) introducing the liquid composition into a fluid bed granulator; (iii) removing solvent to obtain a solid dispersion in particulate form; and (iv) recovering the solid dispersion in particulate form from the fluid bed granulator.

According to the invention, the liquid composition comprising the cannabinoid, the amphiphilic block copolymer and the solvent also comprises the water-soluble film former in at least partially dissolved form.

Further according to the invention, the liquid composition comprising the cannabinoid, the amphiphilic block copolymer and the solvent and the water-soluble film former preferably also comprises the antioxidant in at least partially dissolved form.

The liquid composition may also comprise one or more further excipients. These can be present in any suitable form, for instance, in dissolved form or in dispersed form.

As an example, silicon dioxide can by present in the liquid composition in dispersed form. The cannabidiol and the excipients are present in the liquid compositions in the weight ratios as indicated herein for the pharmaceutical formulations.

The solvent used to prepare the liquid composition can be any solvent capable of at least partially dissolving the cannabinoid, the amphiphilic block copolymer and preferably also the water-soluble film former and/or the antioxidant.

A preferred solvent is ethanol comprising not more than 10% v/v water, such as ethanol comprising not more than 4% v/v water, for instance, ethanol 96% v/v.

As indicated above, the liquid composition is introduced into a fluid bed granulator. In a preferred embodiment, the liquid composition is sprayed into a fluid bed granulator already containing solid particles.

The solid particles contained in the granulator can comprise one or more excipients. In a preferred embodiment, the solid particles comprise a diluent, such as microcrystalline cellulose.

One or more additional excipient, such as colloidal silicon dioxide, can also be present. The fluid bed granulator is operated so that solvent is removed and a solid dispersion in particulate form is obtained. For instance, an inlet air temperature of 45 ± 10°C can be chosen.

Solvent removal can be continued until a predetermined loss on drying (LOD) is reached. For instance, the product can be dried up to loss on drying of not more than 2.0%.

After drying the product is discharged and sieved.

The size of the granules obtained is not limited. Suitable sizes are in the range from 50 µm to 2000 µm, for example in the range from 100 µm to 1000 µm.

Formulations according to the present invention are preferably stable to discoloration. The color remains stable or changes only slightly to off-white upon storage for three months, preferably for six months and in particular for 12 months under long-term conditions (25°C/60% rh).

The granules represent a self-emulsifying solid dispersion. Upon combination with an aqueous medium a micellar solution can be obtained.

A formulation according to the invention, when subjected to an in vitro dissolution test in 0.1N HCI following the USP paddle method, releases at least 60 wt% of the cannabinoid within 60 minutes, preferably at least 90 wt% within 60 minutes, in particular at least 90 wt% within 45 minutes.

The solid dispersion granules can be filled into bottles, sachets or stick packs using commercial standard technology and equipment. The solid dispersion granules are to be sprinkled on food or dispersed in a liquid, *e.g*., water.

A composition obtained by dispersing the solid dispersion granules in a liquid can be applied to patients being not able to swallow by means of a syringe through a gastric tube.

Depending on the final dosage strength per unit, the solid dispersion granules can also be filled into capsules which are feasible for swallowing (*e.g*. capsule size 2-1 for 25 mg/dose). Alternatively, for high dosed units, bigger capsules can be used as a primary packaging material for the granules. Such capsules are not for swallowing (*e.g*. capsule size up to 000 / sprinkle caps for 100-200 mg/dose). Rather, the solid dispersion granules are to be sprinkled on food or dispersed in a liquid, *e.g*., water.

Alternatively, the solid dispersion granules can also be processed into tablets. The solid dispersion granules are combined with one or more excipients, such as a disintegrant, a glidant, and/or a lubricant. The obtained mixture is then compressed into tablets.

In one embodiment, they are processed into orally dispersible tablets.

### Example

The invention is illustrated with the help of a specific example, without being restricted in any way thereby.

### Preparation of granules

Cannabidiol (CBD) granules containing 29.7 % w/w active ingredient are prepared according to the following batch formula:

| Constituent | Function | Quantity (g) | Percentage in the final granulate (%) |
|---|---|---|---|
| CBD | drug substance | 170.78 | 29.7 |
| Poloxamer 188 *(Kolliphor*^{®} *P 188)* | solubilizer | 44.28 | 7.7 |
| Ascorbyl palmitate | antioxidant | 2.30 | 0.4 |
| Microcrystalline Cellulose *(Avicel*^{®} *PH 101)* | diluent | 278.30 | 48.4 |
| Colloidal silicon dioxide *(Aerosil^{®} 200)* | moisture adsorbent | 46.00 | 8.0 |
| Polyvinylpyrrolidone *(Kollidon*^{®} *30)* | binder, solubilizer | 30.48 | 5.3 |
| Silicon Dioxide *(Syloid*^{®} *244 FP Silica)* | moisture adsorbent, glidant | 2.88 | 0.5 |
| Ethanol 96% v/v (a) | granulation liquid | 1150.00 | - |
| **Total theoretical weight** | | 575.02 | 100.0 |

In the first processing step, CBD and the pharmaceutical excipients poloxamer 188, ascorbyl palmitate, microcrystalline cellulose, silicon dioxide, colloidal silicon dioxide and polyvinylpyrrolidone are granulated.

For granulation, the fluid bed granulation technology is used.

The drug substance cannabidiol and the pharmaceutical excipients poloxamer 188, ascorbyl palmitate and polyvinylpyrrolidone are dissolved in ethanol 96% v/v. Silicon dioxide (Syloid^{®} 244 FP) is dispersed in the solution.

Microcrystalline cellulose and colloidal silicon dioxide (Aerosil^{®} 200) are charged into the fluid bed granulator and granulated with the described solution. The granules are discharged and sieved.

The volatile component ethanol 96% v/v is removed from the granules during the drying phase in the fluid bed dryer. The inlet air temperature is 45 ± 10°C, the product temperature 30 - 35°C.

The granules are dried up to a reference value for the loss on drying (LOD) percentage of not more than 2.0%.

### Dosage Form

Cannabidiol granules containing 29.7% w/w cannabidiol are filled in HDPE bottles to provide a total dose of 1500 mg Cannabidiol. The granulate is administered with 240 ml tap water (room temperature) in total. The granulate is firstly dispersed in 100 ml water. The remaining amount of water is used to rinse the container twice.

### Stability of the Cannabidiol Granulate

Samples are stored under accelerated conditions (40°C/75%), under intermediate conditions (30°C/65% rh) and under long-term conditions (25°C/60% rh).

Under storage at accelerated storage conditions the appearance discolored form white to yellowish after one months and to yellow after two months. The color changes only slightly to off-white at long-term conditions after three months and at intermediate conditions after four months.

The dissolution decreases slightly for storage at accelerated conditions after three months but is still well within specification. The dissolution remains unchanged after three months at long-term and after four months at intermediate conditions.

A decrease of assay of about 6 % at accelerated conditions after three months is observed, but the product is still within the shelf-life specification. At intermediate and long-term conditions no significant decrease of assay is observable after four months and three months, respectively.

### Stability of an Aqueous Dispersion

The chemical stability of an aqueous dispersion containing 1500 mg of cannabidiol was tested in a holding time study. For this purpose, about 5 g of a development batch (formulation without Aerosil 200) was dispersed in 240 ml water and stirred at ambient temperature. The impurity profile was monitored for 2 hours.

The impurity profile remains unchanged for the examined time period of two hours. Thus, the dispersion of the product in water for administration will be stable for a time period required for administration.

### CBD Release

Release is tested according to EP 2.9.3 / USP <711>. A paddle dissolution apparatus is used. Dissolution testing is performed at a standard temperature of 37°C ± 0,5°C and a stirrer speed of 100 rpm.

Complete release is observed in 0.1 M HCI + 2% Cetyltrimethylammonium-bromide (CTAB) after 45 min.

## Claims

1. Pharmaceutical formulation in the form of a solid dispersion, wherein the solid dispersion comprises in admixture a cannabinoid, an amphiphilic block copolymer as a solubilizer and a water-soluble film former wherein the components are present in a weight ratio cannabinoid : amphiphilic block copolymer : water soluble film former of 1: 0.16 - 0.36 : 0.08 - 0.28.

2. A formulation according to claim 1, wherein the cannabinoid and the amphiphilic block copolymer are present in a weight ratio cannabinoid: amphiphilic block copolymer of 1 : 0.21 - 0.31.

3. A formulation according to claim 1 or 2, wherein the amphiphilic block copolymer is a block copolymer containing at least one polyoxyethylene block and at least one polyoxypropylene block.

4. A formulation according to claim 3, wherein the amphiphilic block copolymer is a poloxamer, in particular poloxamer 188.

5. A formulation according to any of claims 1 to 4, wherein the cannabinoid and the water soluble film former are present in a weight ratio cannabinoid : water soluble film former of 1 : 0.13 - 0.23.

6. A formulation according to any of claims 1 to 5, wherein the water soluble film former is polyvinylpyrrolidone.

7. A formulation according to any of claims 1 to 5, wherein the water soluble film former is hydroxypropylmethyl cellulose.

8. A formulation according to any of claims 1 to 7, wherein the solid dispersion in addition comprises an antioxidant.

9. A formulation according to claim 8, wherein the antioxidant is used in an amount of 0.5 to 2.5 wt%, preferably of 0.8 to 2 wt%, in particular 1.0 to 1.8 wt%, relative to the amount of the cannabinoid.

10. A formulation according to any of claims 8 and 9, wherein the antioxidant is ascorbyl palmitate.

11. A formulation according to any of claims 1 to 10, wherein the solid dispersion comprises a diluent.

12. A formulation according to claim 11, wherein the cannabinoid and the diluent are present in a weight ratio cannabinoid: diluent of 1:0.5 - 2.7, preferably 1:0.9 - 2.3, in particular 1:1.3 - 1.9.

13. A formulation according to any of claims 11 and 12, wherein the diluent is microcrystalline cellulose.

14. A formulation according to any of claims 1 to 13, wherein the solid dispersion comprises a moisture adsorbent.

15. A formulation according to claim 14, wherein the cannabinoid and the moisture adsorbent are present in a weight ratio cannabinoid : moisture adsorbent of 0.14 - 0.44, preferably 0.19 - 0.39, in particular 0.24 - 0.34.

16. A formulation according to any of claims 14 and 15, wherein the moisture adsorbent comprises a silicon dioxide.

17. A formulation according to any of claims 1 to 16, wherein the solid dispersion is free or essentially free of triglycerides; and/or mono- and diglycerides; and/or fatty acids.

18. A formulation according to any of claims 1 to 17, wherein the cannabinoid is cannabidiol (2-[(1R,6R)-3-methyl-6-(1-methylethenyl)-2-cyclohexen-1-yl]-5-pentyl-1,3-benzenediol).

19. A formulation according to any of claims 1 to 18, wherein the formulation, when subjected to an in vitro dissolution test in 0.1N HCI following the USP paddle method, releases at least 60 wt% of the cannabinoid within 60 minutes, preferably at least 90 wt% within 60 minutes, in particular at least 90 wt% within 45 minutes.

20. A method for preparing a formulation as defined in any of claims 1 to 19, wherein the method comprises the following steps:
(i) preparing a liquid composition comprising the cannabinoid, the amphiphilic block copolymer, the water-soluble film former and a solvent capable of at least partially dissolving the cannabinoid and the amphiphilic block copolymer;
(ii) introducing the liquid composition into a fluid bed granulator;
(iii) removing solvent to obtain a solid dispersion in particulate form; and
(iv) recovering the solid dispersion in particulate form from the fluid bed granulator.

## Patentansprüche

1. Pharmazeutische Formulierung in Form einer festen Dispersion, wobei die feste Dispersion in einer Mischung ein Cannabinoid, ein amphiphiles Blockcopolymer als Lösungsvermittler und einen wasserlöslichen Filmbildner umfasst, wobei die Komponenten in einem Gewichtsverhältnis von Cannabinoid : amphiphiles Blockcopolymer : wasserlöslicher Filmbildner von 1 : 0,16 - 0,36 : 0,08 - 0,28 vorliegen.

2. Formulierung gemäß Anspruch 1, wobei das Cannabinoid und das amphiphile Blockcopolymer in einem Gewichtsverhältnis von Cannabinoid : amphiphiles Blockcopolymer von 1 : 0,21 - 0,31 vorliegen.

3. Formulierung gemäß Anspruch 1 oder 2, wobei das amphiphile Blockcopolymer ein Blockcopolymer ist, das mindestens einen Polyoxyethylenblock und mindestens einen Polyoxypropylenblock enthält.

4. Formulierung gemäß Anspruch 3, wobei das amphiphile Blockcopolymer ein Poloxamer, insbesondere Poloxamer 188, ist.

5. Formulierung gemäß einem der Ansprüche 1 bis 4, wobei das Cannabinoid und der wasserlösliche Filmbildner in einem Gewichtsverhältnis von Cannabinoid : wasserlöslicher Filmbildner von 1 : 0,13 - 0,23 vorliegen.

6. Formulierung gemäß einem der Ansprüche 1 bis 5, wobei der wasserlösliche Filmbildner Polyvinylpyrrolidon ist.

7. Formulierung gemäß einem der Ansprüche 1 bis 5, wobei der wasserlösliche Filmbildner Hydroxypropylmethylcellulose ist.

8. Formulierung nach einem der Ansprüche 1 bis 7, wobei die feste Dispersion zusätzlich ein Antioxidans umfasst.

9. Formulierung nach Anspruch 8, wobei das Antioxidans in einer Menge von 0,5 bis 2,5 Gew.-%, vorzugsweise von 0,8 bis 2 Gew.-%, insbesondere von 1,0 bis 1,8 Gew.-%, bezogen auf die Menge des Cannabinoids, verwendet wird.

10. Formulierung gemäß einem der Ansprüche 8 und 9, wobei das Antioxidans Ascorbylpalmitat ist.

11. Formulierung gemäß einem der Ansprüche 1 bis 10, wobei die feste Dispersion ein Verdünnungsmittel umfasst.

12. Formulierung nach Anspruch 11, wobei das Cannabinoid und das Verdünnungsmittel in einem Gewichtsverhältnis Cannabinoid:Verdünnungsmittel von 1 : 0,5 bis 2,7, vorzugsweise 1 : 0,9 bis 2,3, insbesondere 1 : 1,3 bis 1,9, vorliegen.

13. Formulierung gemäß einem der Ansprüche 11 und 12, wobei das Verdünnungsmittel mikrokristalline Cellulose ist.

14. Formulierung gemäß einem der Ansprüche 1 bis 13, wobei die feste Dispersion ein Feuchtigkeitsadsorbens umfasst.

15. Formulierung gemäß Anspruch 14, wobei das Cannabinoid und das Feuchtigkeitsadsorbens in einem Gewichtsverhältnis Cannabinoid: Feuchtigkeitsadsorbens von 0,14 - 0,44, vorzugsweise 0,19 - 0,39, insbesondere 0,24 - 0,34, vorliegen.

16. Formulierung gemäß einem der Ansprüche 14 und 15, wobei das Feuchtigkeitsadsorbens ein Siliciumdioxid umfasst.

17. Formulierung gemäß einem der Ansprüche 1 bis 16, wobei die feste Dispersion frei oder im Wesentlichen frei von Triglyceriden und/oder Mono- und Diglyceriden und/oder Fettsäuren ist.

18. Formulierung gemäß einem der Ansprüche 1 bis 17, wobei das Cannabinoid Cannabidiol (2-[(1R,6R)-3-Methyl-6-(1-methylethenyl)-2-cyclohexen-1-yl]-5-pentyl-1,3-benzendiol) ist.

19. Formulierung gemäß einem der Ansprüche 1 bis 18, wobei die Formulierung, wenn sie einem In-vitro-Auflösungstest in 0,1 N HCI gemäß dem USP-Paddle-Verfahren unterzogen wird, innerhalb von 60 Minuten mindestens 60 Gew.-% des Cannabinoids, vorzugsweise mindestens 90 Gew.-% innerhalb von 60 Minuten, insbesondere mindestens 90 Gew.-% innerhalb von 45 Minuten freisetzt.

20. Verfahren zur Herstellung einer Formulierung gemäß einem der Ansprüche 1 bis 19, wobei das Verfahren die folgenden Schritte umfasst:
(i) Herstellen einer flüssigen Zusammensetzung, die das Cannabinoid, das amphiphile Blockcopolymer, den wasserlöslichen Filmbildner und ein Lösungsmittel umfasst, das in der Lage ist, das Cannabinoid und das amphiphile Blockcopolymer zumindest teilweise zu lösen;
(ii) Einbringen der flüssigen Zusammensetzung in einen Fließbettgranulator;
(iii) Entfernen des Lösungsmittels, um eine feste Dispersion in Partikelform zu erhalten; und
(iv) Gewinnen der festen Dispersion in Partikelform aus dem Fließbettgranulator.

## Revendications

1. Formulation pharmaceutique sous forme d'une dispersion solide, dans laquelle la dispersion solide comprend en mélange un cannabinoïde, un copolymère séquencé amphiphile comme solubilisant et un agent filmogène hydrosoluble, les composants étant présents dans un rapport pondéral cannabinoïde : copolymère séquencé amphiphile : agent filmogène hydrosoluble de 1 : 0,16 - 0,36 : 0,08 - 0,28.

2. Formulation selon la revendication 1, dans laquelle le cannabinoïde et le copolymère séquencé amphiphile sont présents dans un rapport pondéral cannabinoïde : copolymère séquencé amphiphile de 1 : 0,21 - 0,31.

3. Formulation selon la revendication 1 ou 2, dans laquelle le copolymère séquencé amphiphile est un copolymère séquencé contenant au moins une séquence polyoxyéthylène et au moins une séquence polyoxypropylène.

4. Formulation selon la revendication 3, dans laquelle le copolymère séquencé amphiphile est un poloxamère, en particulier le poloxamère 188.

5. Formulation selon l'une quelconque des revendications 1 à 4, dans laquelle le cannabinoïde et l'agent filmogène hydrosoluble sont présents dans un rapport pondéral cannabinoïde : agent filmogène hydrosoluble de 1 : 0,13 - 0,23.

6. Formulation selon l'une quelconque des revendications 1 à 5, dans laquelle le filmogène hydrosoluble est la polyvinylpyrrolidone.

7. Formulation selon l'une quelconque des revendications 1 à 5, dans laquelle le filmogène hydrosoluble est l'hydroxypropylméthylcellulose.

8. Formulation selon l'une quelconque des revendications 1 à 7, dans laquelle la dispersion solide comprend en outre un antioxydant.

9. Formulation selon la revendication 8, dans laquelle l'antioxydant est utilisé en une quantité de 0,5 à 2,5 % en poids, de préférence de 0,8 à 2 % en poids, en particulier de 1,0 à 1,8 % en poids, par rapport à la quantité de cannabinoïde.

10. Formulation selon l'une quelconque des revendications 8 et 9, dans laquelle l'antioxydant est le palmitate d'ascorbyle.

11. Formulation selon l'une quelconque des revendications 1 à 10, dans laquelle la dispersion solide comprend un diluant.

12. Formulation selon la revendication 11, dans laquelle le cannabinoïde et le diluant sont présents dans un rapport pondéral cannabinoïde:diluant de 1 : 0,5 à 2,7, de préférence de 1 : 0,9 à 2,3, en particulier de 1 : 1,3 à 1,9.

13. Formulation selon l'une quelconque des revendications 11 et 12, dans laquelle le diluant est de la cellulose microcristalline.

14. Formulation selon l'une quelconque des revendications 1 à 13, dans laquelle la dispersion solide comprend un adsorbant d'humidité.

15. Formulation selon la revendication 14, dans laquelle le cannabinoïde et l'adsorbant d'humidité sont présents dans un rapport pondéral cannabinoïde : adsorbant d'humidité de 0,14 à 0,44, de préférence de 0,19 à 0,39, en particulier de 0,24 à 0,34.

16. Formulation selon l'une quelconque des revendications 14 et 15, dans laquelle l'adsorbant d'humidité comprend un dioxyde de silicium.

17. Formulation selon l'une quelconque des revendications 1 à 16, dans laquelle la dispersion solide est exempte ou essentiellement exempte de triglycérides ; et/ou de mono- et diglycérides ; et/ou d'acides gras.

18. Formulation selon l'une quelconque des revendications 1 à 17, dans laquelle le cannabinoïde est le cannabidiol (2-[(1R,6R)-3-méthyl-6-(1-méthyléthényl)-2-cyclohexén-1-yl]-5-pentyl-1,3-benzènediole).

19. Formulation selon l'une quelconque des revendications 1 à 18, dans laquelle la formulation, lorsqu'elle est soumise à un test de dissolution in vitro dans du HCI 0,1 N selon la méthode USP paddle, libère au moins 60 % en poids du cannabinoïde en 60 minutes, de préférence au moins 90 % en poids en 60 minutes, en particulier au moins 90 % en poids en 45 minutes.

20. Procédé de préparation d'une formulation telle que définie dans l'une quelconque des revendications 1 à 19, dans lequel le procédé comprend les étapes suivantes :
(i) préparer une composition liquide comprenant le cannabinoïde, le copolymère séquencé amphiphile, l'agent filmogène hydrosoluble et un solvant capable de dissoudre au moins partiellement le cannabinoïde et le copolymère séquencé amphiphile ;
(ii) introduire la composition liquide dans un granulateur à lit fluidisé ;
(iii) éliminer le solvant pour obtenir une dispersion solide sous forme particulaire ; et
(iv) récupérer la dispersion solide sous forme particulaire à partir du granulateur à lit fluidisé.
